# EUROPEAN PATENT SPECIFICATION

(11) **EP 4 375 339 B1**
(45) Date of publication and mention of the grant of the patent: **02.12.2026**
(21) Application number: 23180021.0
(22) Date of filing: 19.06.2023
(51) Int. Cl.: C09D 5/00, B01J 35/39, C09D 7/63, C09D 7/61, C09D 5/14, B01J 21/06, A61L 9/20, B01J 23/50, B01J 23/72, B01J 23/89

(54) **PHOTOCATALYTIC PASTE BASED ON NATURAL ADHESIVES, POROUS PHOTOCATALYTIC MATERIALS FOR AIR PURIFICATION FROM VOLATILE ORGANIC COMPOUNDS, INORGANIC COMPOUNDS AND MICROORGANISMS, AND A METHOD FOR PRODUCING PHOTOCATALYTIC PASTE BASED ON NATURAL ADHESIVES AND POROUS PHOTOCATALYTIC MATERIALS**
PHOTOKATALYTISCHE PASTE UND VERFAHREN ZUR LUFTREINIGUNG
PATES PHOTOCATALYTIQUES À BASE D'ADHÉSIFS NATURELS ET DE MATÉRIAUX PHOTOCATALYTIQUES POREUX

(30) Priority: 24.11.2022 PL 44293122
(43) Date of publication of application: 29.05.2024
(73) Proprietor: PRODUCENT STOLARKI PCV I AL "BEWI" Bernard Wojcik, 46-060 Zlinice Proszkow (PL); Uniwersytet Gdanski, 80-309 Gdansk (PL); UNIWERSYTET JAGIELLONSKI, 31-007 Kraków (PL)
(72) Inventor: Zaleska-Medynska, Adriana, Gdansk (PL); Bajorowicz, Beata, Gdansk (PL); Golabiewska, Anna, Gdansk (PL); Nadolna, Joanna, Pepowo (PL); Mazierski, Pawel, Pepowo (PL)
(74) Representative: Czarnik, Maciej

(56) References cited:
- EP-A1- 0 792 687
- WO-A1-2021/173021
- JP-A- 2001 139 891
- JP-A- 2001 279 187
- JP-A- 2001 329 220

## Description

The invention refers to paste with photocatalytic properties intended for the purification of air from volatile organic compounds, inorganic compounds, and micro-organisms including viruses and porous materials with a photocatalytic paste layer and a method of to obtaining them. The invention finds application in the removal of volatile organic compounds, inorganic compounds and airborne pathogenic microorganisms such as bacteria, fungi, fungal spores, and viruses. The invention is particularly applicable to deodorisation and purification devices for the photocatalytic removal of micro-organisms and volatile organic compounds in air streams, stationary and portable devices for air purification and deodorisation in enclosed spaces (domestic, office, sports halls, medical facilities), in window devices, in devices incorporated into air-conditioning systems. The invention is applicable to the purification of volatile organic compounds (VOCs), inorganic compounds such as NOx and SO₂, NH₃ and microbial compounds - bacteria, fungi, fungal spores, and viruses.

There are known ways to produce layers with photocatalytic properties by depositing titanium dioxide on a matrix. There are known devices for the purification of air from organic and inorganic odours and micro-organisms, in which a layer with photocatalytic material is mounted.
US 5835840 discloses a system for photocatalytic indoor air purification. A thin layer of titanium dioxide was applied to the ventilation duct's inner surface. The system incorporates lamps emitting ultraviolet light.
US 007255831 discloses a system for photocatalytic air purification in enclosed spaces. It incorporates tungsten(VI) oxide-modified titanium dioxide photocatalyst. The tungsten-modified photocatalyst was obtained by adding a TiO₂ suspension to an aqueous solution. The resulting photocatalyst was then dispersed in water at 25% by weight and applied to the surface of a honeycomb substrate by electrophoretic spraying or dipping. The photocatalyst thin films were fabricated through a homogenisation process of WO₃/TiO₂ nanoparticles in water, which should take between 10 and 30 minutes.
Patent description no. US006797127 discloses a system for air purification that consists of UV light sources. The contaminant degradation process takes place in the presence of an orthorhombic titanium dioxide photocatalyst modified with silver, gold, platinum, tungsten, vanadium or copper. The photocatalyst was deposited on the surface of a metal plate, ceramic substrate, polyester fibre, paper, plastic or paper filter.
Patent description PL223973 discloses a device for the photocatalytic removal of volatile organic contaminants, comprising a plate element on which a photocatalytic layer is applied and a carrier element on which a light source in the form of LEDs emitting UV light, preferably UV-A and/or UV-C, is placed, with the light source being directed towards the photocatalytic layer. The photocatalytic layer is formed by a photocatalyst, consisting of metal-modified titanium dioxide nanotubes. It discloses that the TiO₂-based photocatalytic layer was modified with platinum. The photocatalyst was obtained by adding an aqueous solution of potassium hexachloroplatinate(IV) to alcohol. Afterwards, the titanium dioxide precursor tetra isopropyl titanate (TIP) was added. The resulting sol was dried at 80°C and calcined at 450°C for 3 h. The photocatalyst was then deposited by dipping onto the surface of the ceramic material and placed in the device as a plate element.

The description in invention application P.417116 discloses porous coatings composed of a ceramic deposit or nanotubes, and titanium oxide or titanium oxide modified with platinum and quantum dots. Titanium oxide is applied in paste form by dipping, followed by drying and calcination. TiO₂-Pt is obtained by reduction by introducing K₂PtCl₄ into a suspension of TiO₂ in an alcohol solution, followed by the addition of a reducing agent - NaBH₄. The resulting TiO₂-Pt precipitate is washed with water and ethanol and dried.

Patent description PL.433102 discloses a colloidal grinding system - a paste containing 1 to 60 % by weight (preferably 2 to 36 % by weight) of TiO₂ particles: a mixture of anatase with rutile in a weight ratio of 65:45 to 90:10, with a size of 10⁻⁹ to 10⁻⁵ m, preferably 10⁻⁸ to10⁻⁷ m. The paste also contains a non-ionic surfactant from 1 to 20 % by weight. (preferably 1.6 to 18 % w/w.), which include mixtures of polyoxyethylene derivatives of sorbitan and oleic acid (Tween 80), polyglucoside of coconut oil acids, decyl glucoside, polyethylene glycol (PEG) and p-tert-octylphenol polymer ether (Triton X-100), lauryl glucoside, disodium cocoyl glutamate (Disodium Cocoyl), polyoxyethylene (10) lauryl ether (Laureth-10), polyoxyethylene (23) lauryl ether (Laureth-23), and polyoxyethylene (4) lauryl ether (Laureth-4), preferably p-tert-octylphenol (Triton X-100), the polymer in an amount of 1 to 20 % by weight (preferably 5 to 13% by weight) of the polymer (preferably 5 to 13 % wt.) of the PVP type (poly(vinylpyrrolidone), PEG (poly(ethylene oxide)), PVA (poly(vinyl alcohol)), preferably of the polyether group e.g. poly(ethylene oxide) with a molecular weight of 200 to 20000 (preferably 200 to 1000), a solvent of 40 to 95 % wt., preferably in the range of 65 to 95 % wt. (a polar solvent such as water, methyl alcohol, ethyl alcohol, propyl alcohol, isopropyl alcohol, butyl alcohol, tert-butyl alcohol, preferably isopropanol, ethanol, methanol and mixtures thereof), and a pH correcting agent of 1 to 5 % wt. (preferably 1.5 to 2.7 % wt.) such as hydrogen chloride, nitric acid, sulphuric acid, acetic acid, formic acid, potassium hydroxide, ammonia and mixtures thereof in weight ratios of TiO₂: surfactant: polymer: solvent: pH correcting agent from 0.025:0.025:0.025:1:0.025 to 3:1:1:4.75:0.25, viscosity from 0.001 to 40 Pa·s at 25 °C. It also describes a porous material with photocatalytic properties for air purification from harmful volatile organic compounds, inorganic compounds and microorganisms. Silicon carbide, titanium carbide, tungsten carbide, silicon nitride, boron nitride, titanium nitride, aluminosilicates e.g. halloysite, zirconium oxide, aluminium oxide or a mixture of these with a porosity of 10 to 50 ppi (pores per inch) are used as porous material. Preferably, the thickness of the porous material is between 1 and 50 mm (preferably between 2 and 30 mm), and the porous material is evenly - homogeneously covered by the paste mentioned above. The thickness ratio of the photocatalytic layer to the porous material is between 0.00005:1 and 0.002:1. Beneficial effects are then observed. Preferably, the ratio of the thickness of the paste layer to the thickness of the porous material is chosen so that the thickness of the porous material should be between 0.001 and 0.05 m. Preferably, the thickness of the photocatalytic layer applied to the surface of the porous material should be between 5·10⁻⁸ and 10⁻⁴ m. The implementation examples describe a porous material with a carried photocatalytic paste layer, with a layer thickness range of 5·10⁻⁸ and 10⁻⁴ m and porous material thickness between 0.001 and 0.05 m and 2 and 30 mm. The application describes a method of obtaining a material with photocatalytic properties for air purification through an engineered method of applying pastes to a porous material. A ceramic material such as silicon carbide, titanium carbide, tungsten carbide, silicon nitride, boron nitride, titanium nitride, aluminosilicates e.g. halloysite, zirconium oxide, aluminium oxide or a mixture of these with a porosity of 10 to 50 ppi is used as the porous material.

Patent application JP2001279187 A describes a photocatalyst-containing coating composition for indoor air purification that avoids health and durability problems of conventional systems. The invention replaces organic synthetic binders and solvents with natural, human-harmless binders such as starches, flours, proteins, polysaccharides, and cellulose derivatives, combined with water and a photocatalyst, preferably titanium dioxide. This prevents secondary emission of harmful substances and reduces binder degradation caused by photocatalytic activity. The coating can be applied to interior materials and filters to decompose VOCs such as formaldehyde, toluene, and xylene. Periodic reapplication restores photocatalytic performance and coating integrity.

International patent application WO2021173021 A1 relates to a photocatalytic paste and porous photocatalytic material for purification and deodorisation of air containing volatile organic and inorganic compounds and microorganisms. The paste is a stable colloidal dispersion comprising titanium dioxide particles (anatase, rutile, or mixtures), non-ionic surfactants, polymer binders, a polar solvent, and a pH stabiliser, forming low-toxicity, cost-effective compositions with high adhesion. The paste is applied onto porous ceramic substrates to form durable photocatalytic layers on laboratory and semi-industrial scales. The resulting materials exhibit high photocatalytic activity under low-intensity UV radiation and can be regenerated by UV exposure, restoring purification efficiency during prolonged use in air-treatment systems.

Patent application EP0792687 A1 discloses a photocatalytic functional material that provides deodorizing, antimicrobial, stain-preventing, and harmful-substance decomposition functions while being manufacturable at low temperatures (≤300 °C). The material comprises a photocatalyst (e.g. TiO₂), an electron-trapping metal (such as Cu or Ag), and a photodegradation-resistant matrix (inorganic material or resin). The electron-trapping metal suppresses electron-hole recombination, enabling high photocatalytic activity even when the photocatalyst is embedded in the matrix, which improves mechanical strength and peeling resistance. The invention enables durable coatings on heatsensitive substrates (plastics, films, building materials) with simple coating or curing processes.

From the patent application JP2001139891 A are known photocatalytic materials widely use titanium dioxide (TiO₂) because of its high photocatalytic activity, chemical stability, and non-toxicity. Upon UV irradiation, TiO₂ generates hydroxyl radicals with strong oxidative power, enabling effective decomposition of organic pollutants, odors, microorganisms, and nitrogen oxides. However, conventional photocatalytic systems are difficult to apply to organic substrates, since direct contact between TiO₂ and organic materials leads to oxidative degradation of the substrate. To address this, prior art proposes immobilizing TiO₂ using organic polymer matrices, particularly calcium alginate and related alginate gels with three-dimensional network structures. These polymers encapsulate and fix TiO₂ while protecting organic substrates, and the addition of transition metals (e.g. Cu, Pt) enhances redox activity and photocatalytic efficiency.

The disadvantages of known solutions for photocatalytic materials for air purification are the toxicity of the synthetic adhesives used, usually in the form of non-ionic surfactants (e.g. Triton X-100, poly(vinylpyrrolidone), polyethylene glycol), high cost and toxicity of organic solvents (e.g. ethanol, propyl alcohol, methyl alcohol, isopropyl alcohol, butyl alcohol, tert-butyl alcohol) used to produce photocatalytic layers, the unstable bonding of photocatalyst grains to the matrix surface, and a reduction of photocatalytic activity of the obtained layers in subsequent measurement cycles.

The invention enables to provide paste, porous photocatalytic materials and a method of obtaining photocatalytic paste and porous materials devoid of these disadvantages.

The subject matter of the invention is a qualitatively and quantitatively selected paste composition free of toxic substances and a method of obtaining an air purification material - a porous material with an applied TiO₂-based paste.

The invention is disclosed in the claims.

A quantitative and qualitative composition of the TiO₂-based photocatalytic paste was therefore developed according to the first embodiment used in an amount of 8.85 to 9% wt. of the entire paste. The present invention proposes to use for the production of organic pastes - natural adhesives as a component of the paste, i.e. starch as a single adhesive agent in the paste, especially soluble potato starch, in an amount of 0.9 to 2.2 % wt. to the whole paste, or an aqueous mixture of a ternary adhesion agent - aqueous thre component adhesive agent comprises demineralised water from 61 to 92.9 % wt., preferably 79 % wt., soy protein isolate in a ratio of 5 to 20 % wt., preferably 14 % wt., urotropin in 0.1 to 3 % wt., preferably 2 % wt., and grape extract, in particular Vitis vinifera grape seed extract in a ratio of 2 to 10 % wt., preferably 5 % wt., relative to the total weight of the three-component adhesion agent, this mixture being in a paste in an amount of 0.9 to 2.2% wt.

Also disclosed but not in accordance with the invention is a paste containing a single adhesive ingredient, in addition to the variant above - starch - such as alginate or glyoxal, or maltodextrin, or amylose, or amylopectin, - each ingredient individually in the paste in an amount of from 0.01 to 4.35 % wt. in the total paste. In accordance with the invention the solvent is demineralised water used between 88.5 to 90.1% wt. in the paste.

In accordance with the invention - the paste further comprises silver compounds, or copper compounds, or silver and copper compounds together in an amount of 0.01 to 0.45 % wt. in the whole paste (0.1 % to 5 % wt. relative to TiO₂), which include silver nitrate, silver acetate, silver sulphate, copper(II) acetate, copper(I) acetate, copper(II) nitrate, copper(II) sulphate.

A quantitative and qualitative composition was therefore developed for the photocatalytic paste modified with silver and copper compounds based on TiO₂ with a metal modification of 8.85 to 9 % wt. to the whole paste. The solvent in the paste containing metal or metals is demineralised water used at 88.5 to 90.1 % wt.

Thus, a paste was developed with the use of a natural adhesive component modified with silver/copper compounds. Starch, especially soluble potato starch or an aqueous three-component mixture in the amount of 0.9 to 2.2 % wt., and TiO₂ from 8.85 to 9 % wt. is used as the adhesive agent with the aforementioned metal(s).

Also disclosed but not in accordance with the invention is a method for obtaining a paste not modified with silver and copper compounds comprising the step of mixing by means of a magnetic stirrer at a speed in the range of 200 to 500 rpm, preferably in the range of 300-400 rpm for a time of 30 to 120 min, preferably 30-60 min, titanium dioxide P-25 with a solvent (demineralised water) used in an amount of 91.7 to 99.99 % wt. of the whole paste (the content of TiO₂ alone is from 8.3 % wt. to 9.1 % wt. in the whole paste). Finally, an adhesion agent is introduced: in particular potato soluble starch, in an amount of 0.45 to 4.35 % wt. in the paste, or an aqueous mixture of a three-component adhesion agent comprises demineralised water from 61 to 92.9 % wt., preferably 79 % wt., soy protein isolate at a ratio of 5 to 20 % wt., preferably 14 % wt., urotropine at a ratio of 0.1 to 3 % wt., preferably 2 % wt., to the entire mixture and grape extract, especially Vitis vinifera grape seed extract at 2 to 10 % wt., preferably 5 % wt. relative to the total weight of the three-component adhesion agent, whereby this mixture is introduced into the paste in an amount from 0.45 to 8.3 % wt., or a single adhesion ingredient, in addition to a variant of the aforementioned - starch - such as alginate or glyoxal or maltodextrin or amylose or amylopectin - each ingredient being individually introduced into the paste in an amount from 0.01 % wt. to 4.35 % wt. in the paste.

The method for obtaining a paste modified with silver and copper compounds comprises followng step: mixing by means of a magnetic stirrer at a speed in the range of 200 to 500 rpm, preferably in the range of 300-400 rpm for 30 to 120 min, preferably 30-60 min, titanium dioxide P-25 with a solvent (demineralised water) used in a total amount of 97.35 to 99.09 % wt. in the whole paste (TiO₂ alone constitutes 8.85 to 9 % wt. in the entire paste), then silver and/or copper compounds used in an amount of 0.01 to 0.45 % wt. are introduced. (0.1 to 5 % wt. relative to TiO₂). Finally, an adhesion agent is introduced - in this variant, either starch, especially soluble potato starch, or an aqueous three-component mixture of the aforementioned compounds in an amount of 0.9 to 2.2 % wt. in the entire paste.

The invention also relates to the preparation of a final porous material with photocatalytic properties for the purification of air from harmful volatile organic compounds, inorganic compounds and microorganisms. Silicon carbide, titanium carbide, tungsten carbide, aluminium oxide, and zirconium oxide, with a porosity of 10 to 30 ppi (pores per inch) are used as porous material. The porous material is coated with a paste modified with copper or silver compounds or a copper-silver mixture of 0.1 to 5 % wt. relative to TiO₂. The thickness ratio of the photocatalytic layer to the porous material is between 0.00005:1 and 0.002:1 and the porous material is uniformly - homogeneously coated through the pastes specified above. Preferably, the thickness of the porous material is between 10 and 25 mm, preferably in the 10-21 mm range. Preferably, the thickness of the paste coating of the porous material ranges from 5·10⁻⁸ to 10⁻⁴ m.
The invention is also method for obtaining a final porous material with an applied layer of photocatalytic paste with TiO₂ and either copper or silver compounds or a mixture of copper and silver, involving four steps: (1) the production of a photocatalytic paste of suitable composition - this step is described earlier; (2) the application of the photocatalytic paste to the porous material: silicon carbide, titanium carbide, tungsten carbide, aluminium oxide, zirconia (pore size 10 ppi, 20 ppi or 30 ppi). Prior to deposition of the photocatalytic paste, the porous material surface is cleaned using known agents e.g. dishwashing liquid and water in a volume ratio of e.g. 1:200. The porous materials are then placed in a solvent mixture of water/methanol, water/ethanol, and water/isopropanol in ratios from 1:1 to 1:10, preferably 1:1 to 1:5, and subjected to ultrasounds for 10 to 30 min, preferably 15 min, and dried at 50 to 100°C, preferably between 50 and 80°C, for 10 to 15 h, preferably 12 h. Before dipping the porous material into the photocatalytic paste, the paste is heated to between 30 and 50°C and mixed into a homogeneous mixture using a known method, e.g. on a magnetic stirrer rotating in a range from 100 to 1500 rpm for a period of 5 to 10 min. The paste is then cooled using a known method, e.g. in a water bath, to a temperature of 15 to 20°C - this process prevents the formation of aggregates and produces a homogeneous paste.

The prepared porous material is then immersed in the photocatalytic paste until the porous material are covered by the paste, e.g. one side for 1 to 5 min, preferably for 3 min, then the porous material is turned to the opposite side and immersed in the paste for another 1 to 5 min, preferably for 3 min. In the next step, the excess paste is removed in order to prevent blockage of pores in the porous material, e.g. by blowing the porous material with a stream of inert gas: nitrogen, using a centrifugation method between 1200 and 15000 rpm, preferably 1250 rpm for a period of 5 to 15 min, preferably 5 min.
In step (3), the porous material coated with the photocatalytic paste is dried at a temperature from 50 to 100 °C, preferably in the range of 50-80 °C. In step (4), calcination of the porous material coated with the photocatalytic paste is carried out at a temperature from 400 to 600 °C using a temperature increment from 2 °C/min to 10°C/min, preferably in the range of 2-5 °C/min.

The advantages of the invention are an efficient method for obtaining photocatalytic pastes, highly efficient porous photocatalytic materials for the purification of air from volatile organic compounds, inorganic compounds and microorganisms. The benefits of the invention are the non-toxicity, the availability of ingredients, and the low cost of the reactants used to obtain the photocatalytic paste and thus the porous material. Photocatalytic pastes are homogeneous mixtures that can be easily applied to porous material - resulting in a durable, environmentally friendly, highly effective product.

The invention is described in more detail in the examples and shown in the figures, where:
Fig. 1 presents the results of a degradation efficiency study of toluene for porous materials coated with a photocatalytic paste unmodified with silver and copper compounds with different adhesive agents;
Fig. 2 shows the degradation efficiency of toluene in 10 consecutive measurement cycles in the presence of 0.1%Cu_TiO_{2_}starch porous material (experimental conditions: C₀ₜₒₗᵤₑₙₑ = 50 ppm, exposure time: 30 min);
Fig. 3 shows the surface morphology of porous ceramic materialscoated with a paste containing (a) TiO₂, water and starch, (b-f) a mixture of TiO₂, water, starch, silver and copper compounds;
Fig. 4 shows Raman spectra for a series of porous films coated with pastes modified with silver and copper compounds in amounts ranging from 0.1 to 4 % wt. relative toTiO₂;
Fig. 5 shows UV-Vis absorption spectra of porous films coated with pastes containing: a) TiO₂ nanoparticles, water and different adhesion agent types, b) a mixture of TiO₂ nanoparticles and silver and copper compounds in amounts ranging from 0.1 to 4 % wt. relative to TiO₂.

### Example 1 (Reference example)

### Methodology for obtaining porous films coated with TiO₂ nanoparticles using natural adhesive agents

The process of fabricating porous films coated with TiO₂ nanoparticles using natural adhesive agents consists of four steps: (1) generation of a photocatalytic paste. Table 1 summarises the detailed qualitative and quantitative composition of the seven pastes obtained, which differ in the natural adhesive agent (binder) type used; the composition of the photocatalytic paste (final product) is the same as that added to the mixture during paste preparation; (2) application of the photocatalytic paste to ceramic porous material in the form of a porous sinter made of silicon carbide with a porosity of 30 ppi (pores per inch); (3) drying; and (4) calcination.
(1) In order to obtain a photocatalytic paste with a given composition as specified in Table 1, TiO₂ P25 (a semiconductor with photocatalytic properties) was weighed in a beaker, followed by the addition of a solvent (water). The suspension obtained this way was thoroughly mixed with a magnetic stirrer for 30 min. Afterwards, a natural adhesion agent (amylose, amylopectin, maltodextrin, starch, aqueous mixture: soy protein isolate, urotropin and grape extract, glyoxal or alginate) was introduced into the suspension and mixing continued until a homogeneous mixture was obtained (process conditions: time and rpm are summarised in Table 1).
(2) Once the photocatalytic pastes were produced, they were then applied to porous ceramic material made of silicon carbide, with a pore size of 30 ppi and measuring 2 x 3 x 1 cm. Prior to embedding the photocatalytic paste, the surface of the porous ceramic material was cleaned using "Ludwik" dishwashing liquid and water in a volume ratio of 1:200. The porous material were then placed in a solvent mixture: water/methanol in a ratio of 1:1 and subjected to ultrasound treatment for 15 min and dried at 60 °C for 12 h. Before dipping the porous material into the photocatalytic paste, the paste was heated to 40 °C and stirred on a magnetic stirrer (300 rpm for 5 min). The paste was then cooled in a water bath to 25 °C - this process prevents the formation of aggregates and a homogeneous paste is formed. The prepared ceramic porous material were then immersed in the photocatalytic paste for 3 min, after which the porous sinter was turned over and immersed in the paste for a further 3 min. In the subsequent step, the excess paste was removed by blowing the porous material with a nitrogen jet.
(3) The porous layers thus prepared were dried in an oven at 60 °C for 12 h, then (4) calcined at 450 °C using a temp. increment of 3 °C/min for 2 h to mineralise/remove organic substances and to stably bind the photocatalyst grains on the surface of the porous sinter.

**Table 1. Qualitative and quantitative composition of photocatalytic pastes differing in the type of used natural adhesion agent and the preparation conditions of the photocatalytic layers.**

| Sample name | Paste composition | | Paste components homogenisatio n time, rpm | T (°C) t (h) drying of the layer | T (°C) |
|---|---|---|---|---|---|
| | Ingredient type | Ingredient content in the paste | | | t (h)calcination of the layer |
| TiO₂_amylose | TiO₂ | 10 g | 30 min, 300 rpm | T=60 °C | T=450 °C |
| | H₂O | 100 ml | | t=12 h | t=2 h |
| | binder: amylose | 2.5 g | | | |
| TiO₂_maltodext rin | TiO₂ | 10 g | 30 min, 300 rpm | T=60 °C | T=450 °C |
| | H₂O | 100 ml | | t=12 h | t=2 h |
| | binder: maltodextrin | 2.5 g | | | |
| TiO₂_amylopect in | TiO₂ | 10 g | 30 min, 300 rpm | T=60 °C | T=450 °C |
| | H₂O | 100 ml | | t=12 h | t=2 h |
| | binder: amylopectin | 2.5 g | | | |
| TiO₂_glyoxal | TiO₂ | 10 g | 30 min, 300 rpm | T=60 °C | T=450 °C |
| | H₂O | 100 ml | | t=12 h | t=2 h |
| | binder: glyoxal | 2.5 g | | | |
| TiO_{2_}alginate | TiO₂ | 10 g | 30 min, 300 rpm | T=60 °C | T=450 °C |
| | H₂O | 100 ml | | t=12 h | t=2 h |
| | binder: alginate | 10 mg | | | |
| TiO_{2_}starch | TiO₂ | 10 g | 30 min, 300 rpm | T=60 °C | T=450 °C |
| | H₂O | 100 ml | | t=12 h | t=2 h |
| | binder: starch, water (ratio 1:1) | 2.5 g | | | |
| TiO_{2_}soy | TiO₂ | 10 g | | T=60 °C | T=450 °C |
| | H₂O | 100 ml | 30 min, 400 rpm | t=12 h | t=2 h |
| | binder: soy protein isolate, grape extract, hexamine, water (ratio 6:2:1:34) | 2.5 g | | | |

Further research was aimed at optimising the amount of adhesion agent (based on starch and soy protein isolate) introduced into the photocatalytic paste. Table 2 summarises the obtained photocatalytic layers, which differ in the amount of natural adhesives used to obtain the paste. Modified photocatalytic pastes were prepared according to the procedure described for TiO_{2_}starch and TiO_{2_}soy paste using the amounts of binders specified in Table 2. Further studies determined the impact of the type and amount of natural adhesives added to the paste on the photocatalytic efficiency of the resulting porous layers.

**Table 2. Overview of porous layers containing photocatalytic pastes differing in the type and content of natural adhesion agents.**

| Sample name | Binder | Binder | Paste | T (°C) | T (°C) |
|---|---|---|---|---|---|
| | composition | content in the paste | component homogenisa tion time, rpm | t (h) layer drying | t (h) layer calcination |
| TiO₂_starch_0.5 | starch, water (1:1) | 0.5 g | 30 min, 300 rpm | T=60 °C | T=450 °C |
| | | | | t=12 h | t=2 h |
| TiO_{2_}starch_{_}1 | starch, water (1:1) | 1 g | 30 min, 300 rpm | T=60 °C | T=450 °C |
| | | | | t=12 h | t=2 h |
| TiO_{2_}starch_{_}2.5 | starch, water (1:1) | 2.5 g | 30 min, 300 rpm | T=60 °C | T=450 °C |
| | | | | t=12 h | t=2 h |
| TiO_{2_}starch_{_}5 | starch, water (1:1) | 5 g | 30 min, 300 rpm | T=60 °C | T=450 °C |
| | | | | t=12 h | t=2 h |
| TiO_{2_}soy_{_}0.5 | soy protein isolate, grape extract, hexamine, water (6:2:1:34) | 0.5 g | 30 min, 400 rpm | T=60 °C | T=450 °C |
| | | | | t=12 h | t=2 h |
| TiO_{2_}soy_{_}1 | soy protein isolate, grape extract, hexamine, water (6:2:1:34) | 1 g | 30 min, 400 rpm | T=60 °C | T=450 °C |
| | | | | t=12 h | t=2 h |
| TiO_{2_}soy_{_}2.5 | hexamine, soy protein isolate, grape extract, hexamine, water (6:2:1:34) | 2.5 g | 30 min, 400 rpm | T=60 °C | T=450 °C |
| | | | | t=12 h | t=2 h |
| TiO_{2_}soy_{_}10 | soy protein isolate, grape extract, hexamine, water (6:2:1:34) | 10 g | 30 min, 400 rpm | T=60 °C | T=450 °C |
| | | | | t=12 h | t=2 h |

### Example 2

The methodology for obtaining porous films coated with TiO₂ nanoparticles and compounds of (a) copper or (b) silver or (c) a mixture of copper and silver in an amount of 0.1 % to 4 % by weight relative to TiO₂
The process of producing porous layers coated with TiO₂ nanoparticles and TiO₂ nanoparticles and copper and/or silver compounds consists of four steps: (1) production of a photocatalytic paste of suitable composition; Table 3 summarises the detailed qualitative and quantitative composition of the obtained pastes; (2) application of the photocatalytic paste to ceramic porous material in the form of a porous sinter made of silicon carbide, (3) drying, and (4) calcination. The composition of the photocatalytic paste (final product) is the same as that added to the mixture during paste preparation.
(1) In order to obtain a photocatalytic paste of a specific composition, TiO₂ P25 (a semiconductor with photocatalytic properties) was weighed in a beaker, then a solvent (water) was added and silver and/or copper compounds in the form of nitrates or acetates were introduced (in amounts ranging from 0.1 % to 4 % wt. relative to TiO₂). The suspension obtained this way was thoroughly mixed with a magnetic stirrer for 30 min. A binder was then introduced into the suspension and mixing was continued until obtaining a homogeneous mixture (process conditions: time and rpm are summarised in Table 3).
(2) Once the photocatalytic pastes had been produced, they were then applied to porous ceramic material with a pore size of 30 ppi made of silicon carbide measuring 2 x 3 x 1 cm. Prior to embedding the photocatalytic paste, the surface of the porous ceramic material was cleaned using "Ludwik" dishwashing liquid and water in a volume ratio of 1:200. The porous material were then placed in a solvent mixture: water/methanol in a ratio of 1:1 and subjected to ultrasound treatment for 15 min and dried at 60 °C for 12 h. Before dipping the porous material into the photocatalytic paste, the paste was heated to 40 °C and stirred on a magnetic stirrer (300 rpm for 5 min). The paste was then cooled in a water bath to 25 °C - a process that prevents the formation of aggregates and enables the formation of a homogeneous paste. The prepared ceramic porous materials were then immersed in the photocatalytic paste for 3 min, after which the porous sinter was turned over and immersed in the paste for a further 3 min. In the following step, the excess paste was removed by blowing off the porous material with a nitrogen jet. (3) The porous layers prepared in this way were dried in an oven at 60 °C for 12 h, then (4) calcined at 450 °C using a temp. increment of 3 °C/min for 2 h to mineralise/remove organic substances and to stably bind the photocatalyst grains on the surface of the porous sinter.

**Table 3. Qualitative and quantitative composition of photocatalytic pastes containing TiO₂ nanoparticles and copper and/or silver compounds, and preparation conditions of photocatalytic layers. Samples comprising alginate represent reference examples.**

| Sample name | Paste composition | | Type and amount (% wt.) of metal precursor in relation to TiO₂ | Paste component homogenisation time, rpm | T (°C), t (h) of drying | T (°C), t (h) of calcination |
|---|---|---|---|---|---|---|
| | Ingredient type | Ingredient content in the paste | | | | |
| 1%Ag_T iO₂_algi nate | TiO₂ | 5 g | silver nitrate, 1 % | 1 h, 300 rpm | T=60 °C | T=450 °C |
| | H₂O | 50 ml | | | t=12 h | t=2 h |
| | binder: alginate | 5 mg | | | | |
| | silver nitrate | 0.05 g | | | | |
| 1 %Ag_T iO₂_starch | TiO₂ | 5 g | silver nitrate, 1 % | 1 h, 300 rpm | T=60 °C | T=450 °C |
| | H₂O | 50 ml | | | t=12 h | t=2 h |
| | binder: water, starch (1:1 ratio) | 1.25 g | | | | |
| | silver nitrate | 0.05 g | | | | |
| 1 %Ag_T iO_{2_}soy | TiO₂ | 5 g | silver nitrate, 1 % | 1 h, 400 rpm | T=60 °C | T=450 °C |
| | H₂O | 50 ml | | | t=12 h | t=2 h |
| | binder: soy protein isolate, grape extract, hexamine, water (6:2:1:34) | 0.5 g | | | | |
| | silver nitrate | 0.05 g | | | | |
| 3%Cu_{_}T iO_{2_}algi nate | TiO₂ | 5 g | copper(II) nitrate, 3 % | 1 h, 300 rpm | T=60 °C | T=450 °C |
| | H₂O | 50 ml | | | t=12 h | t=2 h |
| | binder: alginate | 5 mg | | | | |
| | copper(II) nitrate | 0.15 g | | | | |
| 3%Cu_{_}T iO₂_starc h | TiO₂ | 5 g | copper(II) nitrate, 3 % | 1 h, 300 rpm | T=60 °C | T=450 °C |
| | H₂O | 50 ml | | | t=12 h | t=2 h |
| | binder: water, starch (1:1 ratio) | 1.25 g | | | | |
| | copper(II) nitrate | 0.15 g | | | | |
| 3%Cu_{_}T iO_{2_}soy | TiO₂ | 5 g | copper(II) nitrate, 3 % | 1 h, 400 rpm | T=60 °C | T=450 °C |
| | H₂O | 50 ml | | | t=12 h | t=2 h |
| | binder: soy protein isolate, grape extract, hexamine, water (6:2:1:34) | 0.5 g | | | | |
| | copper(II) nitrate | 0.15 g | | | | |
| 0.1%Ag_ TiO₂_so y | TiO₂ | 5 g | silver nitrate, 0.1 % | 1 h, 400 rpm | T=60 °C | T=450 °C |
| | H₂O | 50 ml | | | t=12 h | t=2 h |
| | binder: soy protein isolate, grape extract, hexamine, water (6:2:1:34) | 0.5 g | | | | |
| | silver nitrate | 0.005 g | | | | |
| 0.1%Cu_ TiO₂_soy | TiO₂ | 5 g | copper(II) nitrate, 0.1 % | 1 h, 400 rpm | T=60 °C | T=450 °C |
| | H₂O | 50 ml | | | t=12 h | t=2 h |
| | binder: soy protein isolate, grape extract, hexamine, water (6:2:1:34) | 0.5 g | | | | |
| | copper(II) nitrate | 0.005 g | | | | |
| 0.8%Ag_ TiO₂_soy | TiO₂ | 5 g | silver nitrate, 0.8 % | 1 h, 400 rpm | T=60 °C | T=450 °C |
| | H₂O | 50 ml | | | t=12 h | t=2 h |
| | binder: soy protein isolate, grape extract, hexamine, water (6:2:1:34) | 0.5 g | | | | |
| | silver nitrate | 0.04 g | | | | |
| 1.5%Ag_ TiO₂_so y | TiO₂ | 5 g | silver nitrate, 1.5 % | 1 h, 400 rpm | T=60 °C | T=450 °C |
| | H₂O | 50 ml | | | t=12 h | t=2 h |
| | binder: soy protein isolate, grape extract, hexamine, water (6:2:1:34) | 0.5 g | | | | |
| | silver nitrate | 0.075 g | | | | |
| 1 %Ag_T iO₂_soy_ acetate | TiO₂ | 5 g | silver acetate, 1 % | 1 h, 400 rpm | T=60 °C | T=450 °C |
| | H₂O | 50 ml | | | t=12 h | t=2 h |
| | binder: soy protein isolate, grape extract, hexamine, water (6:2:1:34) | 0.5 g | | | | |
| | silver acetate | 0.05 g | | | | |
| 1.2%Ag_ TiO₂_so y | TiO₂ | 5 g | silver nitrate, 1.2 % | 1 h, 400 rpm | T=60 °C | T=450 °C |
| | H₂O | 50 ml | | | t=12 h | t=2 h |
| | binder: soy protein isolate, grape extract, hexamine, water (6:2:1:34) | | | | | |
| | silver nitrate | 0.06 g | | | | |
| 2%Ag_T iO₂_soy | TiO₂ | 5 g | silver nitrate, 2 % | 1 h, 400 rpm | T=60 °C | T=450 °C |
| | H₂O | 50 ml | | | t=12 h | t=2 h |
| | binder: soy protein isolate, grape extract, hexamine, water (6:2:1:34) | 0.5 g | | | | |
| | silver nitrate | 0.1 g | | | | |
| 2%Cu_T iO₂_soy | TiO₂ | 5 g | copper(II) nitrate, 2 % | 1 h, 400 rpm | T=60 °C | T=450 °C |
| | H₂O | 50 ml | | | t=12 h | t=2 h |
| | binder: soy protein isolate, grape extract, hexamine, water (6:2:1:34) | 0.5 g | | | | |
| | copper(II) nitrate | 0.1 g | | | | |
| 3%Cu_T iO₂_soy_ acetate | TiO₂ | 5 g | copper acetate, 3 % | 1 h, 400 rpm | T=60 °C | T=450 °C |
| | H₂O | 50 ml | | | t=12 h | t=2 h |
| | binder: soy protein isolate, grape extract, hexamine, water (6:2:1:34) | 0.5 g | | | | |
| | copper(II) nitrate | 0.15 g | | | | |
| 0.1%Ag_ TiO₂_starch | TiO₂ | 5 g | silver nitrate, 0.1% | 1 h, 300 rpm | T=60 °C | T=450 °C |
| | H₂O | 50 ml | | | t=12 h | t=2 h |
| | binder: water, starch (1:1 ratio) | 1.25 g | | | | |
| | silver nitrate | 0.005 g | | | | |
| 0.1%Cu_ TiO₂_sta rch | TiO₂ | 5 g | copper(II) nitrate, 0.1 % | 1 h, 300 rpm | T=60 °C | T=450 °C |
| | H₂O | 50 ml | | | t=12 h | t=2 h |
| | binder: water, starch (1:1 ratio) | 1.25 g | | | | |
| | copper(II) nitrate | 0.005 g | | | | |
| 0.8%Ag_ TiO₂_sta rch | TiO₂ | 5 g | silver nitrate, 0.1 % | 1 h, 300 rpm | T=60 °C | T=450 °C |
| | H₂O | 50 ml | | | t=12 h | t=2 h |
| | binder: water, starch (1:1 ratio) | 1.25 g | | | | |
| | silver nitrate | 0.04 g | | | | |
| 1.2%Ag_ TiO₂_sta rch | TiO₂ | 5 g | silver nitrate, 1.2 % | 1 h, 300 rpm | T=60 °C | T=450 °C |
| | H₂O | 50 ml | | | t=12 h | t=2 h |
| | binder: water, starch (1:1 ratio) | 1.25 g | | | | |
| | silver nitrate | 0.06 g | | | | |
| 1.5%Ag_ TiO₂_sta rch | TiO₂ | 5 g | silver nitrate, 0.1 % | 1 h, 300 rpm | T=60 °C | T=450 °C |
| | H₂O | 50 ml | | | t=12 h | t=2 h |
| | binder: water, starch (1:1 ratio) | 1.25 g | | | | |
| | silver nitrate | 0.075 g | | | | |
| 2%Ag_T iO₂_starc h | TiO₂ | 5 g | silver nitrate, 0.1 % | 1 h, 300 rpm | T=60 °C | T=450 °C |
| | H₂O | 50 ml | | | t=12 h | t=2 h |
| | binder: water, starch (1:1 ratio) | 1.25 g | | | | |
| | silver nitrate | 0.1 g | | | | |
| 3.5%Cu_TiO₂_sta rch | TiO₂ | 5 g | copper(II) nitrate, 0.1 % | 1 h, 300 rpm | T=60 °C | T=450 °C |
| | H₂O | 50 ml | | | t=12 h | t=2 h |
| | binder: water, starch (1:1 ratio) | 1.25 g | | | | |
| | copper(II) nitrate | 0.175 g | | | | |
| 4%Cu_T iO₂_starch | TiO₂ | 5 g | copper(II) nitrate, 0.1 % | 1 h, 300 rpm | T=60 °C | T=450 °C |
| | H₂O | 50 ml | | | t=12 h | t=2 h |
| | binder: water, starch (1:1 ratio) | 1.25 g | | | | |
| | copper(II) nitrate | 0.2 g | | | | |
| 0,1%Ag/ 0,1%Cu_ TiO₂_so y | TiO₂ | 5 g | silver nitrate, 0.1 %; copper(II) nitrate, 0.1 % | 1 h, 400 rpm | T=60 °C | T=450 °C |
| | H₂O | 50 ml | | | t=12 h | t=2 h |
| | binder: soy protein isolate, grape extract, hexamine, water (6:2:1:34) | 0.5 g | | | | |
| | silver nitrate | 0.005 g | | | | |
| | copper(II) nitrate | 0.005 g | | | | |
| 1%Ag/3 %Cu_Ti O₂_soy | TiO₂ | 5 g | silver nitrate, 1 %; copper(II) nitrate, 3 % | 1 h, 400 rpm | T=60 °C | T=450 °C |
| | H₂O | 50 ml | | | t=12 h | t=2 h |
| | binder: soy protein isolate, grape extract, hexamine, water (6:2:1:34) | 0.5 g | | | | |
| | silver nitrate | 0.05 g | | | | |
| | copper(II) nitrate | 0.15 g | | | | |
| 0,1%Ag/ 0,1%Cu_ TiO₂_sta rch | TiO₂ | 5 g | silver nitrate, 0.1 %; copper(II) nitrate, 0.1 % | 1 h, 300 rpm | T=60 °C | T=450 °C |
| | H₂O | 50 ml | | | t=12 h | t=2 h |
| | binder: water, starch (1:1 ratio) | 0.5 g | | | | |
| | silver nitrate | 0.005 g | | | | |
| | copper(II) nitrate | 0.005 g | | | | |
| 1%Ag/3 %Cu_Ti O₂_starc h | TiO₂ | 5 g | silver nitrate, 1 %; copper(II) nitrate, 3 % | 1 h, 300 rpm | T=60 °C | T=450 °C |
| | H₂O | 50 ml | | | t=12 h | t=2 h |
| | binder: water, starch (1:1 ratio) | 0.5 g | | | | |
| | silver nitrate | 0.05 g | | | | |
| | copper(II) nitrate | 0.15 g | | | | |

### Example 3 (Reference example)

Study of the efficiency of toluene removal from the gas phase in a closed photoreactor in the presence of TiO₂-coated porous layers obtained in the presence of natural bonding agents

### Description of the test set-up and measurement procedure:

The photocatalytic efficiency of the resulting porous layers was investigated in the degradation reaction of toluene in the gaseous phase under the influence of radiation emitted by LEDs. The measurement system consisted of a stainless steel photoreactor with a volume of 35 cm³, equipped with two ball valves, a sampling port and a quartz window that separates the radiation source (UV LEDs) from the reaction space. Porous photocatalytic layers measuring 2 x 3 cm were placed at the bottom of the photoreactor. The reactor was then filled with a mixture of air and toluene with an initial concentration of toluene between 25 and 100 ppm. The test system was kept in the dark for 30 min to establish adsorption-desorption equilibrium, and then irradiated for 30 min. Gas samples of 200 µl were taken after 5, 10, 15 and 30 min using a gas-tight syringe, and the concentration of toluene in the collected samples was analysed using a gas chromatograph by Chromatografie Shimadzu 2013 with an FID flame ionisation detector. Nitrogen was used as the carrier gas.

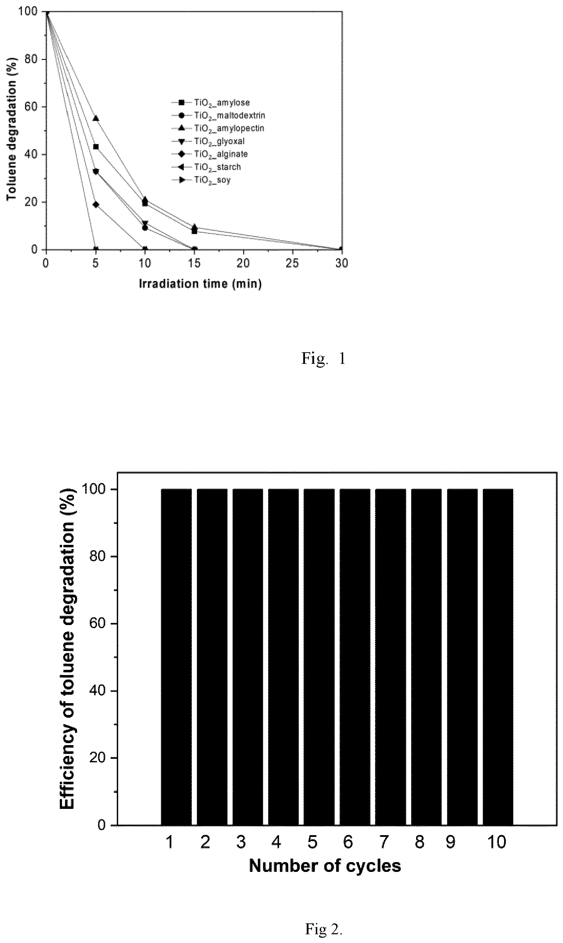

The impact of the type of natural bonding agents on the photocatalytic activity of the resulting porous layers was investigated. The results of the photodegradation efficiency in the presence of porous layers obtained using different bonding agents of natural origin (the method of obtaining these layers is described in Example 1) are shown in Fig. 1 and summarised in Table 4. It was shown that photocatalytic layers obtained in the presence of starch, alginate or soy protein had the highest photocatalytic activity (100 % degradation of toluene after 10 min of illumination). Additionally, all samples showed 100 % photodegradation efficiency of toluene after 30 min. Further studies aimed to optimise the amount of selected bonding agents (based on starch and soy protein isolate) introduced into the photocatalytic paste (Table 4). As a result of the performed work considering ecological and economic aspects, the two most efficient samples were chosen: photocatalytic layers obtained using (1) a paste containing TiO₂ and starch in a ratio of 10:2.5 and (2) a paste containing TiO₂ and a soy-based bonding agent (soy protein isolate, urotropine, hexamine) in a ratio of 10:1.

**Table 4. Results of the photodegradation efficiency of toluene in the presence of porous films coated with TiO₂ nanoparticles obtained in the presence of natural bonding agents.**

| Sample name | Adhesive type | Initial toluene concentration | Toluene degradation efficiency after 10 min | Toluene degradation efficiency after 30 min |
|---|---|---|---|---|
| TiO₂_amylose | amylose | 100 ppm | 81 % | 100 % |
| TiO₂_maltodextrin | maltodextrin | 100 ppm | 91 % | 100 % |
| TiO₂_amylopectin | amylopectin | 100 ppm | 79 % | 100 % |
| TiO₂_glyoxal | glyoxal | 100 ppm | 89 % | 100 % |
| TiO₂_alginate | alginate | 100 ppm | 100 % | 100 % |
| TiO₂_starch_0.5 | starch | 100 ppm | 93 % | 100 % |
| TiO₂_starch_1 | starch | 100 ppm | 75 % | 100 % |
| TiO₂_starch_2.5 | starch | 100 ppm | 100 % | 100 % |
| TiO₂_starch_5 | starch | 100 ppm | 81 % | 100 % |
| TiO₂_soy_0.5 | soy protein isolate, urotropin, grape extract | 100 ppm | 91 % | 100 % |
| TiO₂_soy_1 | soy protein isolate, urotropin, grape extract | 100 ppm | 100 % | 100 % |
| TiO₂_soy_2.5 | soy protein isolate, urotropin, grape extract | 100 ppm | 100 % | 100 % |
| TiO₂_soy_10 | soy protein isolate, urotropin, grape extract | 100 ppm | 100% | 100 % |

### Example 4

Study of the efficiency of toluene removal from the gaseous phase in a closed photoreactor in the presence of porous layers coated with TiO₂ and silver and/or copper compounds obtained in the presence of natural bonding agents

Further work included the modification of photocatalytic pastes based on water and natural binding agents with silver and/or copper compounds. The detailed preparation conditions are described in Example 2, and the results of the toluene photodegradation in the presence of the obtained porous layers are summarised in Table 5. The highest photocatalytic efficiency, i.e. 100 % degradation of toluene after 10 min of irradiation was achieved in the presence of photocatalytic layers containing copper compounds at 0.1 % wt. in relation to TiO₂: 0.1%Cu_{_}TiO_{2_}starch; 0.1%Cu_{_}TiO_{2_}soy, photocatalytic layers containing silver compounds at 0.1 % and 0.8 % wt.: 0.1%Ag_{_}TiO_{2_}starch; 0.8%Ag_{_}TiO_{2_}starch; and a photocatalytic layer containing both silver (0.1 % wt.) and copper (0.1 % wt.) compounds: 0,1%Cu/0,1%Ag_{_}TiO_{2_}starch.

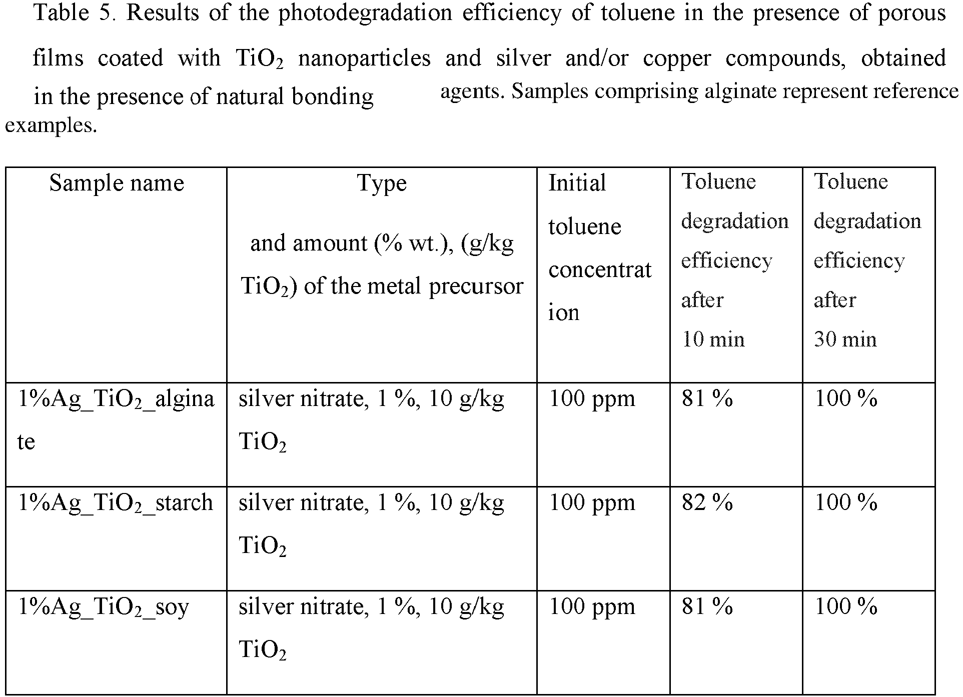

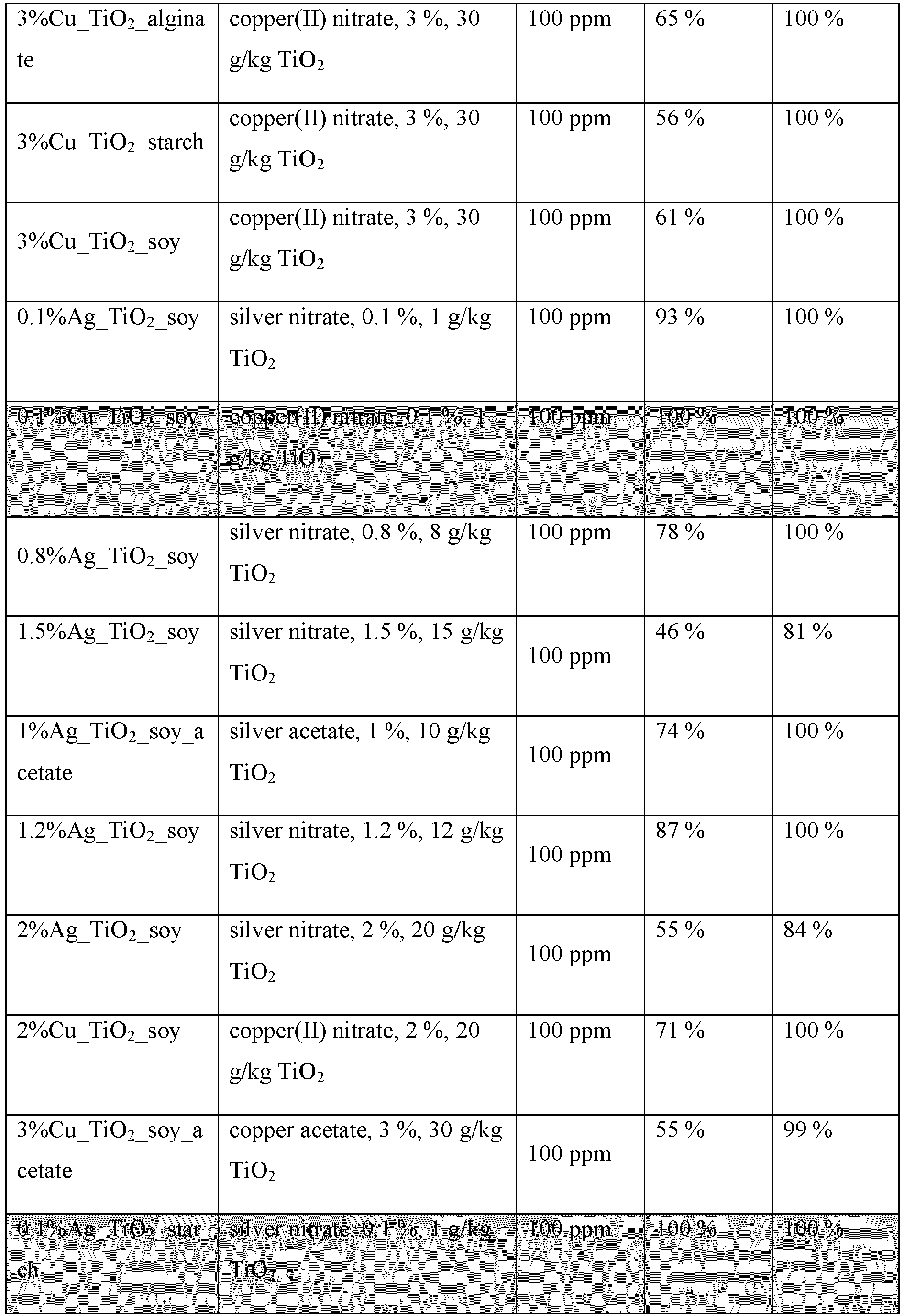

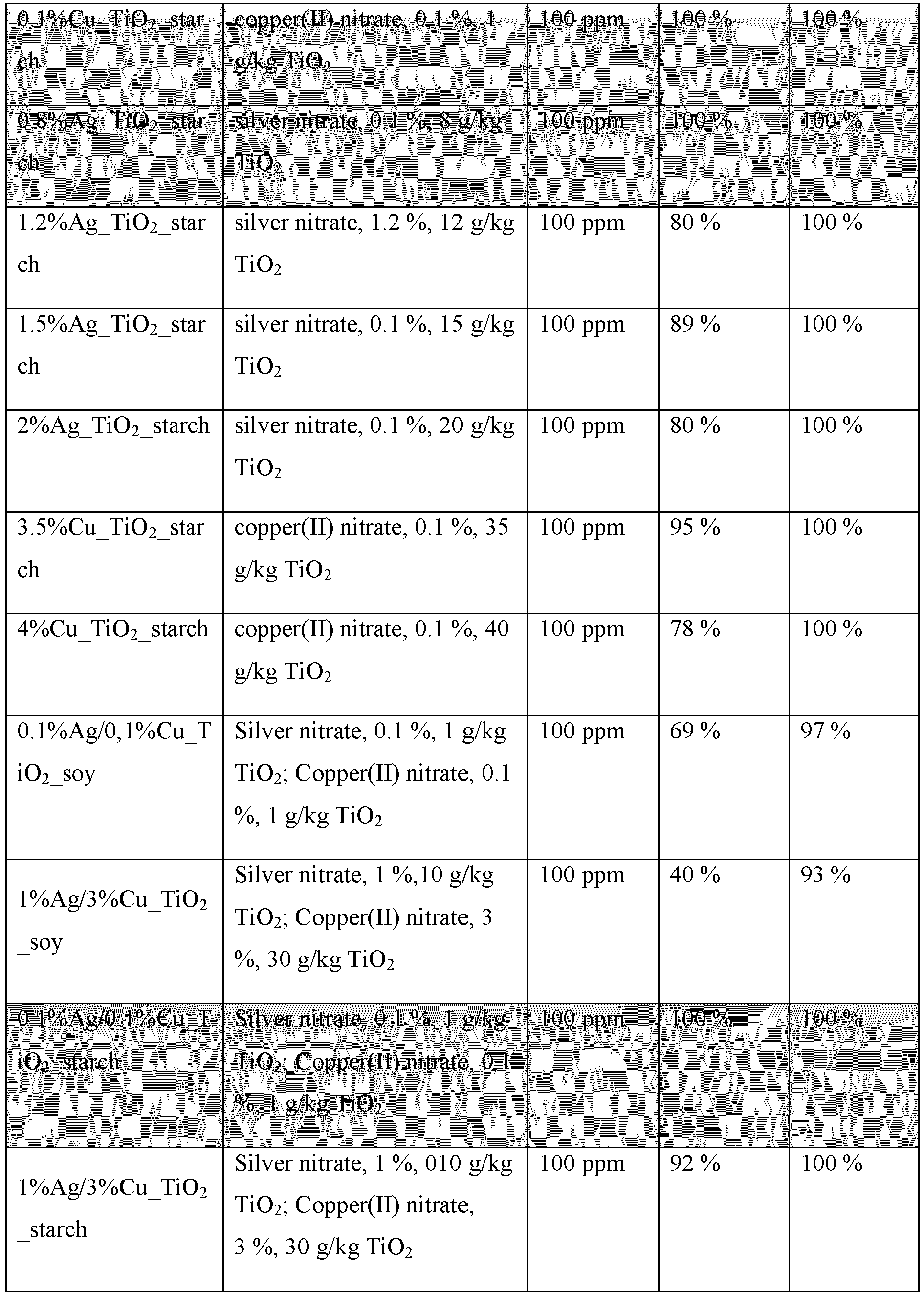

The subsequent stage of the work studied the effects of selected parameters: (i) calcination temperature (ranging from 400 to 500 °C), (ii) calcination time (2 h or 6 h), (iii) pore size of the porous sinter (10, 20 or 30 ppi), (iv) initial toluene concentration (25, 50 or 100 ppm) on the photocatalytic efficiency of the selected porous layer: 0.1%Cu_{_}TiO_{2_}starch (Table 6). It can be seen that the use of a lower calcination temperature (400 °C) results in a reduction in the photocatalytic activity of the selected porous layer - the photodegradation efficiency of toluene, in this case, was 87 % after 10 min of exposure (regardless of the calcination time used: 2 h or 6 h). The results suggest that a higher calcination temperature (450 °C or 500 °C) should be used for complete mineralisation/removal of organic matter and for stable bonding of the photocatalyst grains on the surface of the porous sinter. Furthermore, the obtained results show that changing the pore size of the porous sinter and the initial toluene concentration does not change the photocatalytic activity of the selected photocatalytic layer - the photodegradation efficiency of toluene in the presence of the 0.1%Cu_{_}TiO_{2_}starch sample was in each case 100 % after 10 min of LED irradiation.

**Table 6. Results of toluene photodegradation efficiency in the presence of a 0.1%Cu_{_}TiO_{2_}starch porous layer - effect of toluene's initial concentration, temperature and calcination time of the porous layers and pore size of the porous sinter.**

| Sample name | Porous ceramics pore size (ppi) | T (°C) t (h) calcination | Initial toluene concentration | Toluene degradation efficiency after 10 min | Toluene degradation efficiency after 30 min |
|---|---|---|---|---|---|
| 0.1%Cu_{_}TiO_{2_} starch | 10 | T=450 °C | 100 ppm | 100 % | 100 % |
| | | t=2 h | | | |
| | 20 | T=450 °C | 100 ppm | 100 % | 100 % |
| | | t=2 h | | | |
| | 30 | T=450 °C | 100 ppm | 100 % | 100 % |
| | | t=2 h | | | |
| | 30 | T=450 °C | 50 ppm | 100 % | 100 % |
| | | t=2 h | | | |
| | 30 | T=450 °C | 25 ppm | 100 % | 100 % |
| | | t=2 h | | | |
| | 30 | T=400 °C | 100 ppm | 87 % | 100 % |
| | | t=2 h | | | |
| | 30 | T=500 °C | 100 ppm | 100 % | 100 % |
| | | t=2 h | | | |
| | 30 | T=450 °C | 100 ppm | 100 % | 100 % |
| | | t=6 h | | | |
| | 30 | T=400 °C | 100 ppm | 87 % | 100 % |
| | | t=6 h | | | |
| | 30 | T=500 °C | 100 ppm | 100 % | 100 % |
| | | t=6 h | | | |

### Example 5

### Assessment of the photocatalytic layer's photostability

Assessment of the photocatalytic layer's photostability involved the use of the test system and chromatographic analysis described in Example 3. For each measurement cycle, the photoreactor was filled with a mixture of air and toluene with an initial concentration of 50 ppm, the test system was kept in the dark for 30 min to establish adsorption-desorption equilibrium, and then illuminated for 30 min. Gaseous samples of 200 µl were taken after 5 and 30 min. Ten consecutive measurement cycles were carried out.

The photocatalytic stability of a selected porous layer: 0.1%Cu_{_}TiO_{2_}starch was tested in 10 consecutive measurement cycles in a model reaction for the photodegradation of toluene in the gaseous phase. The results of toluene photodegradation efficiency are shown in Fig. 2. The resulting porous layer was shown to have very good photocatalytic stability over time - the photocatalytic activity in the presence of the test sample was 100 % after 30 min of illumination and remained unchanged during 10 consecutive measurement cycles.

### Example 6 Morphology and physicochemical properties of the obtained photocatalytic layers

The surface morphology of the obtained photocatalytic films was tested using a JEOL high-resolution field emission scanning electron microscope (model JSM-7610F). Crystal structure analysis of the obtained photocatalytic films was conducted through Raman spectroscopy using a Thermo Scientific DXR2 Smart Raman spectrometer (laser wavelength: 532 nm, laser power: 8 mW). Testing of optical properties was carried out using a UV-Vis spectrophotometer by Thermo Scientific (model Evolution 220).

### Surface morphology

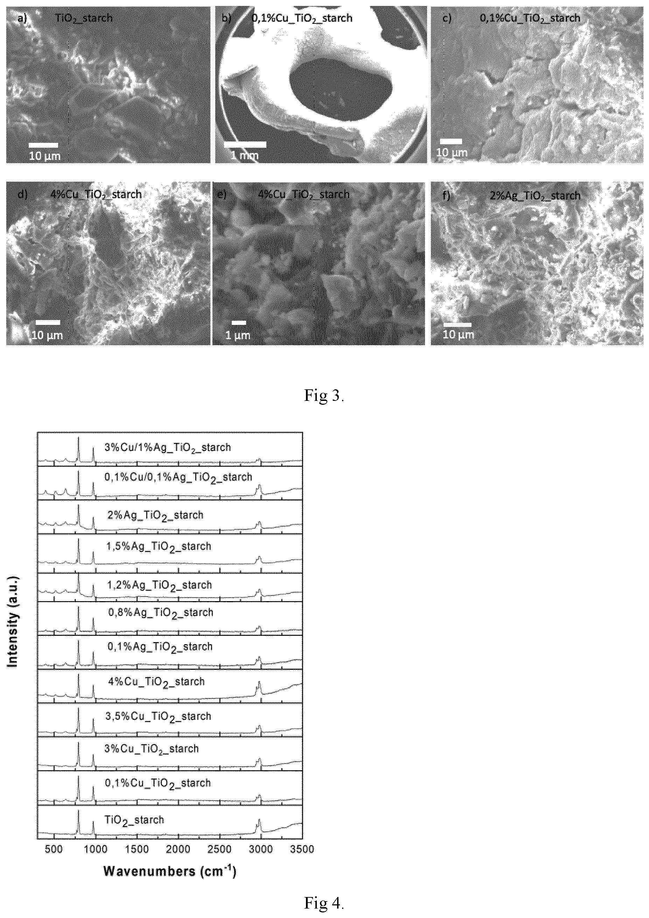

The surface morphology of the selected samples was determined using an SEM scanning electron microscope. SEM images are presented in Fig. 3a-f. Fig. 3a shows a x1,600 magnified image of a porous layer coated with a paste containing TiO₂, water and starch (TiO_{2_}starch). It can be seen that the particles have different shapes and sizes. The particles in the image correspond to µm-sized agglomerates, some of which are even smaller (nm size). Fig. 3b shows an image at low magnification of x25 for the 0.1%Cu_{_}TiO_{2_}starch sample, where an overall image of the pore size of the sintered porous photocatalytic layer (30 ppi) can be seen. Fig. 3c shows the image at a higher magnification of x 2,200, where irregular particles of µm and nm size can be observed. For the 4%Cu_{_}TiO₂ sample, observations were made at low (fig. 3d) and high magnification (fig. 3e) to observe the surface of the agglomerates. The surfaces of agglomerates feature small particles visible in a lighter colour. The morphology of the porous materials - known as a spatially shaped moulding coated with a paste containing (i) TiO₂, water and starch and (*ii*) a mixture of TiO₂, water, starch, silver and/or copper compounds was characterised by an irregular distribution of particles of different shapes and sizes, with irregularly distributed small bright particles observable on the agglomerate surfaces.

### Raman Spectroscopy

Fig. 4 presents Raman spectra for a series of porous films coated with pastes modified with silver and copper compounds in amounts ranging from 0.1 to 4 % wt. relative to TiO₂. Visible sharp bands at 796, 966 and 2989 cm⁻¹ characteristic of silicon carbide (the porous ceramic layer material) were observed for all presented samples. The bands at 396, 518 and 638 cm⁻¹ correspond to the stretching and bending vibrations of the O-Ti-O and Ti-O groups for TiO₂ in the anatase form.

### Optical properties

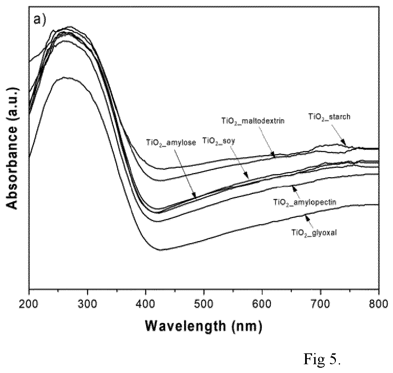

Fig. 5. presents UV-Vis absorption spectra of porous films coated with pastes containing (a) TiO₂ nanoparticles, water and different adhesive agent types, (b) a mixture of TiO₂ nanoparticles and silver and copper compounds in amounts ranging from 0.1 to 4 % wt. relative to TiO₂. For all presented samples, a sharp collapse of the absorption bands was observed at wavelengths in the 400-410 nm range. The characteristic absorption band for TiO₂ was observed from 200 to 410 nm. In addition, the results shown in Fig. 5a indicate that the optical properties of porous films depend on the type of adhesion agent used. The highest intensity of light absorption in the range of 200-800 nm was exhibited by the sample obtained using starch, while the lowest light absorption capacity was exhibited by the porous photocatalytic layer obtained using glyoxal as a bonding agent. Fig. 5b presents the UV-Vis absorption spectra of porous photocatalytic films containing TiO₂ nanoparticles and silver and/or copper compounds. It can be observed that samples modified with silver nanoparticles at 0.8 % and 1 % wt. of TiO₂ had the highest absorption intensity in the 200-800 nm range.

## Claims

1. A photocatalytic paste based on unmodified TiO₂ for air purification from microorganisms comprising titanium dioxide, a solvent, a non-ionic surfactant as an adhesive agent, **characterised in that** it comprises from 8.85 to 9% wt. of paste of TiO₂ particles with a grain size of 10⁻⁹ to 10⁻⁵ m and a structure of anatase, rutile or a mixture thereof, and the adhesive agent is in the form of:
a starch, preferably soluble potato starch in an amount of 0.9 to 2.2% wt. of the paste, or
an aqueous three-component mixture comprising 61 to 92.9% wt. demineralised water, preferably 79% wt., soy protein isolate in a ratio of 5 to 20% wt., preferably 14% wt., urotropin in a ratio of 0.1 to 3% wt., preferably 2% wt., and a grape extract, preferably in a form of extract from *Vitis vinifera* grape seeds in a ratio of 2 to 10% wt., preferably 5% wt., in relation to the total weight of the three-component adhesive agent, this aqueous three-component mixture being in the paste in an amount of 0.9 to 2.2% wt. with the solvent being demineralised water used in an amount of from 88.5 to 90.1% wt. in the paste,
wherein it comprises a silver compound or a copper compound or silver-copper compounds in an amount of 0.1% to 5% wt. relative to TiO₂.

2. The paste according to claim 1, wherein it comprises silver nitrate or silver acetate or silver sulphate or copper(II) acetate, or copper(I) acetate, or copper(II) nitrate, or copper(II) sulphate.

3. The paste according to claim 1-2, wherein it comprises silver compounds or copper compounds or compounds of both silver and copper in an amount of 0.01 to 0.45% wt. in the whole paste, which include: silver nitrate, silver acetate, silver sulphate, copper(II) acetate, copper(I) acetate, copper(II) nitrate, copper(II) sulphate.

4. A porous material in the form shaped moulding with photocatalytic properties for purification of air from microorganisms, volatile organic compounds, and inorganic compounds; the porous material being evenly coated with a layer with photocatalytic properties, **characterised in that** as the porous material silicon carbide or titanium carbide, or tungsten carbide, or aluminium oxide, or zirconium oxide is used with a porosity of 10 to 30 ppi pores per inch and the porous material is evenly coated with the paste described according in claims 1-3 to form a layer with photocatalytic properties and the thickness ratio of the photocatalytic layer to the porous material is between 0.00005:1 and 0.002:1

5. The porous material according to claim 4 wherein the thickness of the porous material is between 10 and 25 mm, preferably in the range of 10-21 mm.

6. The porous material according to claim 4, wherein the thickness of the porous material coating with paste forming a layer is between 5·10⁻⁸ and 10⁻⁴ m.

7. A method for obtaining paste modified with silver and copper compounds according to any claim form 1 to 3 is performed by mixing with a magnetic stirrer at a speed in the range of 200 to 500 rpm, preferably in the range of 300-400 rpm for a period of 30 to 120 min, preferably 30-60 min, titanium dioxide with a solvent in the form of demineralised water used in an amount of 97.35 to 99.09% wt. in the whole paste of the entire paste using TiO₂ alone in an amount of 8.85 to 9% wt. in the entire paste, followed by the addition of silver and/or copper compounds used in an amount of 0.01 to 0.45% wt. of the paste and finally an adhesive agent is added in an amount of 0.9 to 2.2% wt. in the form of starch, in particular soluble potato starch or a three-component aqueous mixture of the adhesive agent comprises demineralised water in an amount of 61 to 92.9% wt., preferably 79% by weight, soy protein isolate at a ratio of 5 to 20% wt., preferably 14% wt., urotropin at a ratio of 0.1 to 3% wt., preferably 2% wt., to the three-component mixture, and grape extract, especially Vitis vinifera grape seed extract at a ratio of 2 to 10% wt., preferably 5% wt., to the total weight of the three-component adhesive agent aqueous mixture.

8. A method for obtaining a porous material with photocatalytic properties in the form of shaped moulding with photocatalytic properties for purifying air of microorganisms, volatile organic compounds, inorganic compounds, whereby the porous material is evenly coated with a layer featuring photocatalytic properties, characterised that the four steps are performed: (1) producing the photocatalytic paste described in claims 1-3; (2) applying a photocatalytic paste to a porous material as described in claims 4-6 and prior to application of the photocatalytic paste, cleaning the surface of the porous material by known means; the material is then placed in a mixture of solvents and exposed to an ultrasonic bath for a period of 10 to 30 min, and dried at a temperature between 50 and 100°C, then prior to application the paste is heated to between 30 and 50°C, after which the paste is cooled by known means to between 15 and 20 °C, then the prepared porous material is immersed in the photocatalytic paste until it is covered and the excess paste is removed at the next step; (3) drying the photocatalytic paste coated porous material at a temperature of 50 to 100°C; (4) calcination of the porous material coated with the photocatalytic paste is carried out at a temperature between 400°C and 600°C using a temperature increase of 2°C/min to 10°C/min preferably between 2°C and 5°C/min.

## Patentansprüche

1. Photokatalytische Paste auf Basis von unmodifiziertem TiO₂ zur Reinigung der Luft von Mikroorganismen, umfassend Titandioxid, ein Lösungsmittel und ein nichtionisches Tensid als Haftmittel, **dadurch gekennzeichnet, dass** sie 8,85 bis 9 Gew.-% der Paste an TiO₂-Partikeln mit einer Korngröße von 10⁻⁹ bis 10⁻⁵ m und einer Anatas-, Rutil- oder einer Mischstruktur davon umfasst, und dass das Haftmittel in Form von:
eine Stärke, vorzugsweise löslicher Kartoffelstärke, in einer Menge von 0,9 bis 2,2 Gew.-% der Paste, oder einem wässrigen Dreikomponentengemisch, umfassend 61 bis 92,9 Gew.-% demineralisiertes Wasser, vorzugsweise 79 Gew.-%, Sojaproteinisolat in einem Anteil von 5 bis 20 Gew.-%, vorzugsweise 14 Gew.-%, Urotropin in einem Anteil von 0,1 bis 3 Gew.-%, vorzugsweise 2 Gew.-%, und einen Traubenextrakt, vorzugsweise in Form eines Traubenkernextrakts aus *Vitis vinifera*, in einem Anteil von 2 bis 10 Gew.-%, vorzugsweise 5 Gew.-%, bezogen auf das Gesamtgewicht des Dreikomponenten-Haftmittels, vorliegt, wobei das wässrige Dreikomponentengemisch in der Paste in einer Menge von 0,9 bis 2,2 Gew.-% mit einem Lösungsmittel enthalten ist, wobei das Lösungsmittel demineralisiertes Wasser ist, das in einer Menge von 88,5 bis 90,1 Gew.-% der Paste verwendet wird,
wobei es eine Silberverbindung oder eine Kupferverbindung oder Silber-Kupfer-Verbindungen in einer Menge von 0,1 bis 5 Gew.-%, bezogen auf TiO₂, umfasst.

2. Paste nach Anspruch 1, **dadurch gekennzeichnet, dass** sie Silbernitrat oder Silberacetat oder Silbersulfat oder Kupfer(II)-acetat oder Kupfer(I)-acetat oder Kupfer(II)-nitrat oder Kupfer(II)-sulfat umfasst.

3. Paste nach einem der Ansprüche 1 bis 2, **dadurch gekennzeichnet, dass** sie Silberverbindungen oder Kupferverbindungen oder Verbindungen sowohl von Silber als auch von Kupfer in einer Menge von 0,01 bis 0,45 Gew.-% bezogen auf die gesamte Paste umfasst, wobei diese umfassen: Silbernitrat, Silberacetat, Silbersulfat, Kupfer(II)-acetat, Kupfer(I)-acetat, Kupfer(II)-nitrat und Kupfer(II)-sulfat.

4. Poröses Material in Form eines Formkörpers mit photokatalytischen Eigenschaften zur Reinigung der Luft von Mikroorganismen, flüchtigen organischen Verbindungen und anorganischen Verbindungen, wobei das poröse Material gleichmäßig mit einer Schicht mit photokatalytischen Eigenschaften beschichtet ist, **dadurch gekennzeichnet, dass** als poröses Material Siliziumcarbid oder Titancarbid oder Wolframcarbid oder Aluminiumoxid oder Zirkoniumoxid mit einer Porosität von 10 bis 30 Poren pro Zoll (ppi) verwendet wird und das poröse Material gleichmäßig mit der gemäß einem der Ansprüche 1 bis 3 beschriebenen Paste beschichtet ist, um eine Schicht mit photokatalytischen Eigenschaften zu bilden, wobei das Dickenverhältnis der photokatalytischen Schicht zur Dicke des porösen Materials zwischen 0,00005:1 und 0,002:1 liegt.

5. Poröses Material nach Anspruch 4, **dadurch gekennzeichnet, dass** die Dicke des porösen Materials zwischen 10 und 25 mm, vorzugsweise zwischen 10 und 21 mm, liegt.

6. Poröses Material nach Anspruch 4, **dadurch gekennzeichnet, dass** die Dicke der Beschichtung des porösen Materials mit der die Schicht bildenden Paste zwischen 5 × 10⁻⁸ und 10⁻⁴ m liegt.

7. Verfahren zur Herstellung einer mit Silber- und Kupferverbindungen modifizierten Paste nach einem der Ansprüche 1 bis 3, **dadurch gekennzeichnet, dass** es durch Mischen von Titandioxid mit einem Lösungsmittel in Form von demineralisiertem Wasser, das in einer Menge von 97,35 bis 99,09 Gew.-% der gesamten Paste verwendet wird, unter Verwendung eines Magnetrührers bei einer Geschwindigkeit im Bereich von 200 bis 500 U/min, vorzugsweise im Bereich von 300 bis 400 U/min, über einen Zeitraum von 30 bis 120 min, vorzugsweise 30 bis 60 min, durchgeführt wird, wobei TiO₂ allein in einer Menge von 8,85 bis 9 Gew.-% der gesamten Paste verwendet wird, woraufhin Silber- und/oder Kupferverbindungen in einer Menge von 0,01 bis 0,45 Gew.-% der Paste zugegeben werden und schließlich ein Haftmittel in einer Menge von 0,9 bis 2,2 Gew.-% in Form von Stärke, insbesondere löslicher Kartoffelstärke, oder eines wässrigen Dreikomponentengemisches des Haftmittels zugegeben wird, wobei das Haftmittelgemisch demineralisiertes Wasser in einer Menge von 61 bis 92,9 Gew.-%, vorzugsweise 79 Gew.-%, Sojaproteinisolat in einem Anteil von 5 bis 20 Gew.-%, vorzugsweise 14 Gew.-%, Urotropin in einem Anteil von 0,1 bis 3 Gew.-%, vorzugsweise 2 Gew.-%, bezogen auf das Dreikomponentengemisch, und Traubenextrakt, insbesondere Traubenkernextrakt aus *Vitis vinifera*, in einem Anteil von 2 bis 10 Gew.-%, vorzugsweise 5 Gew.-%, bezogen auf das Gesamtgewicht des wässrigen Dreikomponenten-Haftmittelgemisches, umfasst.

8. Verfahren zur Herstellung eines porösen Materials mit photokatalytischen Eigenschaften in Form eines Formkörpers mit photokatalytischen Eigenschaften zur Reinigung der Luft von Mikroorganismen, flüchtigen organischen Verbindungen und anorganischen Verbindungen, wobei das poröse Material gleichmäßig mit einer Schicht mit photokatalytischen Eigenschaften beschichtet wird, **dadurch gekennzeichnet, dass** es die folgenden vier Schritte umfasst: (1) Herstellen der in den Ansprüchen 1 bis 3 beschriebenen photokatalytischen Paste; (2) Aufbringen der photokatalytischen Paste auf ein poröses Material gemäß den Ansprüchen 4 bis 6 und, vor dem Aufbringen der photokatalytischen Paste, Reinigen der Oberfläche des porösen Materials mit bekannten Mitteln; anschließend wird das Material in eine Mischung von Lösungsmitteln eingebracht und für einen Zeitraum von 10 bis 30 min einem Ultraschallbad ausgesetzt und bei einer Temperatur zwischen 50 und 100°C getrocknet; vor dem Aufbringen wird die Paste auf eine Temperatur zwischen 30 und 50°C erwärmt, anschließend durch bekannte Mittel auf eine Temperatur zwischen 15 und 20°C abgekühlt; danach wird das vorbereitete poröse Material in die photokatalytische Paste eingetaucht, bis es mit der Paste bedeckt ist, und der überschüssige Teil der Paste wird im folgenden Schritt entfernt; (3) Trocknen des mit der photokatalytischen Paste beschichteten porösen Materials bei einer Temperatur von 50 bis 100°C; (4) Kalzinieren des mit der photokatalytischen Paste beschichteten porösen Materials bei einer Temperatur zwischen 400°C und 600°C, wobei die Temperatur mit einer Erhöhungsgeschwindigkeit von 2°C/min bis 10°C/min, vorzugsweise von 2°C/min bis 5°C/min, erhöht wird.

## Revendications

1. Pâte photocatalytique à base de TiO₂ non modifié pour la purification de l'air des microorganismes, comprenant du dioxyde de titane, un solvant, un tensioactif non ionique en tant qu'agent adhésif, **caractérisée en ce qu'**elle comprend de 8,85 à 9 % en poids de la pâte de particules de TiO₂ ayant une granulométrie de 10⁻⁹ à 10⁻⁵ m et présentant une structure d'anatase, de rutile ou un mélange de celles-ci, et **en ce que** l'agent adhésif se présente sous la forme :
d'un amidon, de préférence de l'amidon de pomme de terre soluble, en une quantité de 0,9 à 2,2 % en poids de la pâte, ou
d'un mélange aqueux à trois composants comprenant de 61 à 92,9 % en poids d'eau déminéralisée, de préférence 79 % en poids, de l'isolat de protéines de soja dans une proportion de 5 à 20 % en poids, de préférence 14 % en poids, de l'urotropine dans une proportion de 0,1 à 3 % en poids, de préférence 2 % en poids, et un extrait de raisin, de préférence sous la forme d'un extrait de pépins de raisin de *Vitis vinifera*, dans une proportion de 2 à 10 % en poids, de préférence 5 % en poids, par rapport au poids total de l'agent adhésif à trois composants, ledit mélange aqueux à trois composants étant présent dans la pâte en une quantité de 0,9 à 2,2 % en poids, le solvant étant de l'eau déminéralisée utilisée en une quantité de 88,5 à 90,1 % en poids de la pâte,
et **en ce qu'**elle comprend un composé d'argent ou un composé de cuivre ou des composés d'argent et de cuivre en une quantité de 0,1 à 5 % en poids par rapport au TiO₂.

2. Pâte selon la revendication 1, **caractérisée en ce qu'**elle comprend du nitrate d'argent, ou de l'acétate d'argent, ou du sulfate d'argent, ou de l'acétate de cuivre(II), ou de l'acétate de cuivre(I), ou du nitrate de cuivre(II), ou du sulfate de cuivre(II).

3. Pâte selon l'une quelconque des revendications 1 et 2, **caractérisée en ce qu'**elle comprend des composés d'argent ou des composés de cuivre ou des composés à la fois d'argent et de cuivre en une quantité de 0,01 à 0,45 % en poids de la pâte totale, lesdits composés comprenant : du nitrate d'argent, de l'acétate d'argent, du sulfate d'argent, de l'acétate de cuivre(II), de l'acétate de cuivre(I), du nitrate de cuivre(II) et du sulfate de cuivre(II).

4. Matériau poreux sous la forme d'une pièce moulée présentant des propriétés photocatalytiques pour la purification de l'air des microorganismes, des composés organiques volatils et des composés inorganiques, le matériau poreux étant uniformément revêtu d'une couche présentant des propriétés photocatalytiques, **caractérisé en ce que** le matériau poreux utilisé est du carbure de silicium, ou du carbure de titane, ou du carbure de tungstène, ou de l'oxyde d'aluminium, ou de l'oxyde de zirconium, présentant une porosité de 10 à 30 pores par pouce (ppi), et **en ce que** le matériau poreux est uniformément revêtu de la pâte décrite selon l'une quelconque des revendications 1 à 3, afin de former une couche présentant des propriétés photocatalytiques, le rapport d'épaisseur de la couche photocatalytique à l'épaisseur du matériau poreux étant compris entre 0,00005:1 et 0,002:1.

5. Matériau poreux selon la revendication 4, **caractérisé en ce que** l'épaisseur du matériau poreux est comprise entre 10 et 25 mm, de préférence entre 10 et 21 mm.

6. Matériau poreux selon la revendication 4, **caractérisé en ce que** l'épaisseur du revêtement du matériau poreux par la pâte formant ladite couche est comprise entre 5-10⁻⁸ et 10⁻⁴ m.

7. Procédé d'obtention d'une pâte modifiée par des composés d'argent et de cuivre selon l'une quelconque des revendications 1 à 3, **caractérisé en ce qu'**il est réalisé par mélange, à l'aide d'un agitateur magnétique à une vitesse comprise entre 200 et 500 tr/min, de préférence entre 300 et 400 tr/min, pendant une durée de 30 à 120 min, de préférence de 30 à 60 min, du dioxyde de titane avec un solvant sous la forme d'eau déminéralisée utilisée en une quantité de 97,35 à 99,09 % en poids de la pâte totale, le TiO₂ étant utilisé seul en une quantité de 8,85 à 9 % en poids de la pâte totale, puis par addition de composés d'argent et/ou de cuivre utilisés en une quantité de 0,01 à 0,45 % en poids de la pâte, et enfin par addition d'un agent adhésif en une quantité de 0,9 à 2,2 % en poids, sous la forme d'amidon, notamment d'amidon de pomme de terre soluble, ou d'un mélange aqueux à trois composants, l'agent adhésif comprenant de l'eau déminéralisée en une quantité de 61 à 92,9 % en poids, de préférence 79 % en poids, de l'isolat de protéines de soja dans une proportion de 5 à 20 % en poids, de préférence 14 % en poids, de l'urotropine dans une proportion de 0,1 à 3 % en poids, de préférence 2 % en poids, par rapport au mélange à trois composants, et un extrait de raisin, notamment un extrait de pépins de raisin de *Vitis vinifera*, dans une proportion de 2 à 10 % en poids, de préférence 5 % en poids, par rapport au poids total du mélange aqueux à trois composants constituant l'agent adhésif.

8. Procédé d'obtention d'un matériau poreux présentant des propriétés photocatalytiques sous la forme d'une pièce moulée présentant des propriétés photocatalytiques pour la purification de l'air des microorganismes, des composés organiques volatils et des composés inorganiques, dans lequel le matériau poreux est uniformément revêtu d'une couche présentant des propriétés photocatalytiques, **caractérisé en ce qu'**il comprend les quatre étapes suivantes : (1) production de la pâte photocatalytique décrite dans l'une quelconque des revendications 1 à 3 ; (2) application de la pâte photocatalytique sur un matériau poreux tel que décrit dans l'une quelconque des revendications 4 à 6, et, avant l'application de la pâte photocatalytique, nettoyage de la surface du matériau poreux par des moyens connus ; le matériau est ensuite placé dans un mélange de solvants et soumis à un bain à ultrasons pendant une durée de 10 à 30 min, puis séché à une température comprise entre 50 et 100 °C ; avant l'application, la pâte est chauffée à une température comprise entre 30 et 50 °C, puis refroidie par des moyens connus à une température comprise entre 15 et 20 °C ; ensuite, le matériau poreux ainsi préparé est immergé dans la pâte photocatalytique jusqu'à ce qu'il en soit recouvert, puis l'excès de pâte est éliminé à l'étape suivante ; (3) séchage du matériau poreux revêtu de la pâte photocatalytique à une température comprise entre 50 et 100 °C ; (4) calcination du matériau poreux revêtu de la pâte photocatalytique à une température comprise entre 400 °C et 600 °C, avec une vitesse d'augmentation de la température de 2 °C/min à 10 °C/min, de préférence de 2 °C/min à 5 °C/min.
